# EUROPEAN PATENT APPLICATION

(11) **EP 4 414 392 A1**
(43) Date of publication of application: **14.08.2024**
(21) Application number: 22878381.7
(22) Date of filing: 28.09.2022
(51) Int. Cl.: C08F 20/10

(54) **CARBONATE (METH)ACRYLATE COMPOSITION**

(30) Priority: 06.10.2021 JP 2021164448
(71) Applicant: NOF Corporation, Shibuya-ku Tokyo 150-6019 (JP)
(72) Inventor: AONO Tatsuya, Amagasaki-shi Hyogo 660-0095 (JP); TAGAMI Yasunobu, Amagasaki-shi Hyogo 660-0095 (JP)
(74) Representative: Mewburn Ellis LLP
(86) International application number: PCT/JP2022/036075
(87) International publication number: WO 2023/058511

(57) **Abstract**

An object is to provide a carbonate (meth)acrylate composition excellent in stability of color phase after storage at a high temperature and polymerizability after storage at a high temperature.

A carbonate (meth)acrylate composition contains 99 to 99.99 mass% of carbon (meth)acrylate and 0.01 to 1 mass% of an ascorbic acid derivative represented by formula (1). 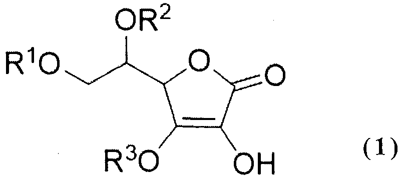 (In the formula (1), R¹, R² and R³ independently represent hydrogen atom, alkyl group having a carbon number of 1 to 20 or acyl group having a carbon number of 1 to 20, respectively.)

## Description

### TECHNICAL FIELD

The present invention is related to a carbonate (meth)acrylate composition.

### BACKGROUND ARTS

It is known that a (meth)acrylate monomer with carbonate group has high polarity and high agglomeration force derived from carbonate group. It is disclosed that anti-drug property, heat resistance and high transparency are exhibited by introducing the (meth)acrylate monomer having carbonate group and that it is applicable as a coating material of a display material (Patent document 1).

Further, as a method of producing the monomer, it is known the method of reacting an epoxy compound and carbon dioxide and it is reported that the (meth)acrylate monomer having carbonate group produced by this method is colorless and transparent (Patent document 2).

Although the (meth)acrylate monomer with carbonate group has high transparency and adhesion as described above, the denaturing of the monomer having carbonate group at a high temperature had become problematic. For example, when it is transported, the temperature may exceed 40°C in summer depending on the storage condition, so that the denaturing of the monomer takes place. Specifically, although the purity of the (meth)acrylate monomer with carbonate group stored at a high temperature of 40°C or higher is not substantially changed before and after the storage, considerable coloration may occur. Such coloration provides an inhibiting factor for the adjustment of color phase in an application of a paint requiring the transparency. It has been expected the development of (meth)acrylate monomer with carbonate group which is resistant to coloration during the storage. Further, in the case that such (meth)acrylate monomer having carbonate group is stored at a high temperature of 40°C or higher and the monomer is used to perform the polymerization, it is observed the phenomenon that the molecular weight of the polymer becomes small. The adjustment of the molecular weight of the polymer is thereby difficult, inhibiting the application of the monomer in fields, such as display application, requiring the precise control of the molecular weight.

As to the deterioration of color phase of the monomer described above, it is said to be effective to add polymerization inhibitor or oxidation prevention agent. For example, it is reported that the stability of storage of the monomer is improved by adding tocopherol to glycerol (meth)acrylate (Patent document 3).

### [PRIOR TECHNICAL DOCUMENTS]

### [PATENT DOCUMENTS]

[PATENT DOCUMENT 1] Japanese Patent Publication No. 2002-287352 A
[PATENT DOCUMENT 2] Japanese Patent Publication No. 2021-506808 A
[PATENT DOCUMENT 3] Japanese Patent Publication No. 2007-509849 A

### SUMMARY OF THE INVENTION

### [OBJECT TO BE SOLVED BY THE INVENTION]

However, according to the composition of patent document 3, although the possibility of the suppression of turbidity is confirmed, it is not described the suppression of coloration of the monomer after the storage at a high temperature or suppression of reduction of the molecular weight in the case of polymerization.

Based on such background, it is demanded carbonate group containing monomer in which coloration after the storage at a high temperature is suppressed, the stability of the color phase is excellent and the reduction of the polymerizability during the polymerization after the storage at a high temperature is suppressed to result in precise control of the molecular weight.

An object of the present invention is to provide a carbonate (meth)acrylate composition excellent in stability of color phase after storage at a high temperature and polymerizability after the storage at a high temperature.

### [SOLUTION FOR THE OBJECT]

As the inventors researched for solving the problems, it is found that the above problems can be solved by carbonate group-containing (meth)arylate composition including an ascorbic acid derivative having a specific structure.

That is, the present invention is as follows.
[1] A carbonate (meth)acrylate composition comprising 99 to 99.99 mass% of a carbonate (meth)acrylate and 0.01-1 mass% of an ascorbic acid derivative represented by a following formula (1), 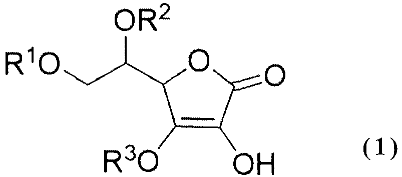 (In said formula (1),
   R¹, R² and R³ independently represent hydrogen atom, alkyl group having a carbon number of 1 to 20, or acyl group having a carbon number of 1 to 20, respectively.).
(2) The carbonate (meth)acrylate composition of (1), wherein said carbonate (meth) acrylate is represented by a following formula (2), formula (3) or formula (4), 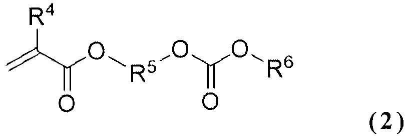 (In the formula (2),
   R⁴ represents hydrogen atom or methyl group,
   R⁵ represents alkylene group having a carbon number of 1 to 10, and
   R⁶ represents hydrogen atom or alkyl group having a carbon number of 1 to 20.) 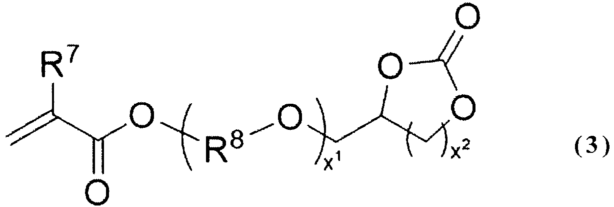 (In the formula (3),
      R⁷ represents hydrogen atom or methyl group,
      R⁸ represents alkylene group having a carbon number of 1 to 10,
      X¹ represents 0 or 1, and
      X² represents 0 to 2.) 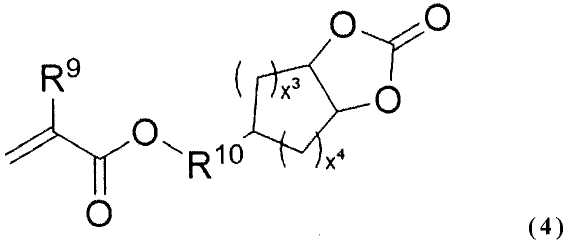 (In the formula (4),
         R⁹ represents hydrogen atom or methyl group,
         R¹⁰ represents alkylene group having a carbon number of 1 to 10,
         X³ represents 0 to 2, and
         X⁴ represents 0 to 1.).

### [EFFECT OF THE INVENTION]

According to the present invention, it is possible to provide a carbonate (meth)acrylate compound excellent in the stability of color phase after the storage at a high temperature and polymerizability after the storage at a high temperature.

### EMBODIMENTS FOR CARRYING OUT THE INVENTION

The embodiments of the present invention will be described below.

### (Carbonate (meth)acrylate (A))

The carbonate (meth)acrylate of the present invention is a monomer including a carbonate structural unit and a meth(acrylate) structural unit. The carbonate structural unit means the structural unit of -O-(C=O)-O-. The carbonate structure may preferably be cyclic carbonate structure and more preferably be 5-membered ring, 6-membered ring or 7-membered ring carbonate structure.

According to the present invention, (meth)acryl is a generic term including acryl and methacryl, and (meth)acrylate structural unit is an acrylate or methacrylate structural unit.

According to a preferred embodiment, the carbonate (meth)acrylate (A) is a compound represented by the following general formula (2), (3) or (4).

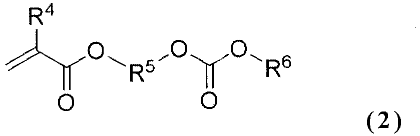

In the formula (2), R⁴ represents hydrogen atom or methyl group.

R⁵ represents alkylene group having a carbon number of 1 to 10, and the carbon number may preferably be 1 or higher, may preferably be 4 or lower, more preferably be 2 or 3, and most preferably be 2.

R⁶ represents hydrogen atom or alkyl group having a carbon number of 1 to 20. The carbon number of the alkyl group of R⁶ may preferably be 1 or higher, preferably be 10 or lower, more preferably be 1 or higher and 6 or lower, and most preferably be 1 or higher and 4 or lower.

The carbonate (meth)acrylate (A) represented by formula (2) may be, for example, (2-methoxycarbonyl)oxyethyl acrylate, (2-methoxycarbonyl)oxyethyl methacrylate, (2-methoxycarbonyl)oxybutyl methacrylate, (2-ethoxycarbonyl)oxyethyl methacrylate, (2-butoxycarbonyl)oxyethyl methacrylate or the like.

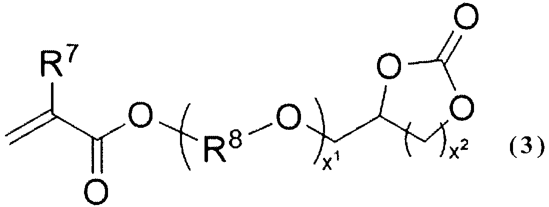

In the formula (3), R⁷ represents hydrogen atom or methyl group.

R⁸ represents alkylene group having a carbon number of 1 to 10, and the carbon number is preferably 1 or higher, is preferably 6 or lower, is preferably 2 or higher and 4 or lower, and more preferably 4.

X¹ represents 0 or 1, and X² represents 0 to 2. X² is preferably 1 to 2 and more preferably 1.

The carbonate (meth)acrylate (A) represented by formula (3) may be, for example, (2-oxo-1,3-dioxolan-4-yl) methyl acrylate, (2-oxo-1,3-dioxolan-4-yl) methyl methacrylate or 4-[(2-oxo-1,3-dioxolan-4-yl) methoxy] butyl acrylate.

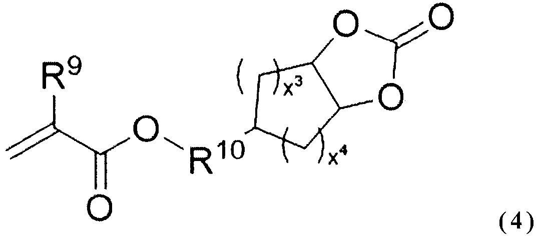

In the formula (4), R⁹ represents hydrogen atom or methyl group, and methyl group is preferred.

R¹⁰ represents alkylene group having a carbon number of 1 to 10, and the carbon number may preferably be 1 or higher, preferably be 4 or lower, preferably be 1 or higher and 3 or lower, and 1 is more preferred.

X³ represents 0 to 2, may preferably be 1 to 2, and 2 is more preferred. X⁴ represents 0 to 1 and 1 is preferred,

The carbonate (meth)acrylate (A) represented by the formula (4) may be, for example, hexahydro-2-oxo-1,3-benzodioxole-5-yl) methyl methacrylate.

Among them, as the cyclic carbonate has reactivity with amine or the like, (2-oxo-1,3-dioxolan-4-yl) methyl methacrylate, and hexahydro-2-oxo-1,3-benzodioxole-5-yl) methyl methacrylate are preferred. Among them, (2-oxo-1,3-dioxolan-4-yl) methyl methacrylate is particularly preferred.

One kind of the carbonate (meth)acrylate (A) may be applied alone or the two kinds or more may be applied in combination.

The content of the carbonate (meth)acrylate is made 99 to 99.99 mass%, when the total content of the carbonate (meth)acrylate and the ascorbic acid derivative represented by the formula (1) is made 100 mass%. Within this range, it is possible to obtain a carbonate (meth)acrylate composition having good stability of color phase and good stability of polymerizability.

On such viewpoint, the content of the carbonate (meth)acrylate may preferably be 99.5 mass% or higher. Further, the content of the carbonate (meth)acrylate is made 99.99 mass% or lower and is particularly preferably 99.95 mass% or lower.

### (Ascorbic acid derivative represented by formula (1))

The ascorbic acid derivative applied in the present invention is a compound represented by the following formula (1).

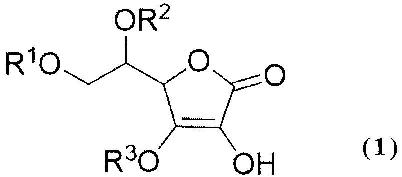

In the formula (1), R¹, R² and R³ represents independently hydrogen atom, alkyl group having a carbon number of 1 to 20, or acyl group having a carbon number of 1 to 20.

Here, R¹ is preferably hydrogen atom, alkyl group or acyl group each having a carbon number of 12 to 20, is more preferably hydrogen atom or acyl group having a carbon number of 12 to 20, more preferably acyl group having a carbon number of 12 to 18.

R² is preferably hydrogen atom, or alkyl group or acyl group each having a carbon number of 1 to 4, is preferably hydrogen atom or acyl group having a carbon number of 1 to 4, and more preferably hydrogen atom.

R³ is preferably hydrogen atom, alkyl group or acyl group each having a carbon number of 1 to 4, more preferably hydrogen atom or alkyl group having a carbon number of 1 to 4, and is more preferably hydrogen atom.

These alkyl group and acyl group may be of straight-chain or branched chain and is preferably of straight-chain.

The ascorbic acid derivative represented by the formula (1) may be, for example, L-ascorbic acid, 3-O-ethyl-L-ascorbic acid, 6-O-palmitoyl-L-ascorbic acid or the like, and 6-O-palmitoyl-L-ascorbic acid is particularly preferred, on the viewpoint of stability of color phase and compatibility.

One kind of the ascorbic acid derivative represented by the formula (1) may be applied alone or the two or more kinds may be applied in combination.

As the total content of the carbonate (meth)acrylate and the ascorbic acid derivative represented by the formula (1) is defined as 100 mass%, the content of the ascorbic acid derivative represented by the formula (1) is the remainder of the content of the carbonate (meth)acrylate.

### (Polymerization inhibitor)

A polymerization inhibitor may be optionally added to the composition of the present invention. The blending ratio of the polymerization inhibitor may preferably be 0.001 to 0.1 mass parts and more preferably be 0.001 to 0.01 mass parts, with respect to 100 mass parts of the carbonate (meth)acrylate.

### EXAMPLES

The present invention will be described further in detail, referring to the inventive and comparative examples.

### (Inventive examples 1 to 11 and comparative examples 1 to 6)

The carbonate (meth)acrylate (component (A)) shown in table 1, the ascorbic acid derivative (component (B)) represented by the formula (1) and shown in table 2, and a comparative coloring preventing agent (component (B')) were mixed according to the blending ratios shown in tables 3, 4 and 5 to provide the respective compositions.

### (Evaluation of stability of color phase after storage at 60°C)

150g of the carbonate (meth)acrylate composition prepared as described in tables 3, 4 and 5 was weighed and heated at 60°C for 3 hours. The Platinum-Cobalt Color Scales (APHA) of the respective carbonate (meth)acrylate compositions were measured by " OME-2000" manufactured by Nippon Denshoku Industries Co., Ltd.. The evaluation was made based on the thus obtained values.
"Ω": less than 100
"O": 100 or more and less than 200
"Δ": 200 or more and less than 300
"×": 300 or more

**Table 1**

| Component (A) (carbonate (meth)acrylate) | | Formula (2) | | | Formula (3) | | | | Formula (4) | | | |
|---|---|---|---|---|---|---|---|---|---|---|---|---|
| | | R4 | R5 | R6 | R7 | R8 | X1 | X2 | R9 | R10 | X3 | X4 |
| A1 | 2-methoxycarbonyloxy ethyl acrylate | H | C2H4 | CH3 | | | | | | | | |
| A2 | 2-methoxycarbonyloxy ethyl methacrylate | CH3 | C2H4 | CH3 | | | | | | | | |
| A3 | 2-butoxycarbonyloxy ethyl methacrylate | CH3 | C2H4 | C4H9 | | | | | | | | |
| A4 | (2-oxo-1,3-dioxolan-4-yl) methyl acrylate | | | | H | | 0 | 1 | | | | |
| A5 | (2-oxo-1,3-dioxolan-4-yl) methyl methacrylate | | | | CH3 | | 0 | 1 | | | | |
| A6 | 4-[(2-oxo-1,3-dioxolan-4-yl) methoxy] butyl acrylate | | | | H | C4H9 | 1 | 1 | | | | |
| A7 | (hexahydro-2-oxo-1,3-benzodioxole-5-yl) methyl methacrylate | | | | | | | | CH3 | CH2 | 2 | 1 |

**Table 2**

| Component (B) (ascorbic acid derivative of formula (1) | | 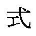 (1) | | |
|---|---|---|---|---|
| | | R1 | R2 | R3 |
| B1 | L-ascorbic acid | H | H | H |
| B2 | 6-O-palmitoyl-L-ascorbic acid | C15H31C(O) | H | H |
| B3 | 3-O-ethyl-L-ascorbic acid | H | H | C2H5 |

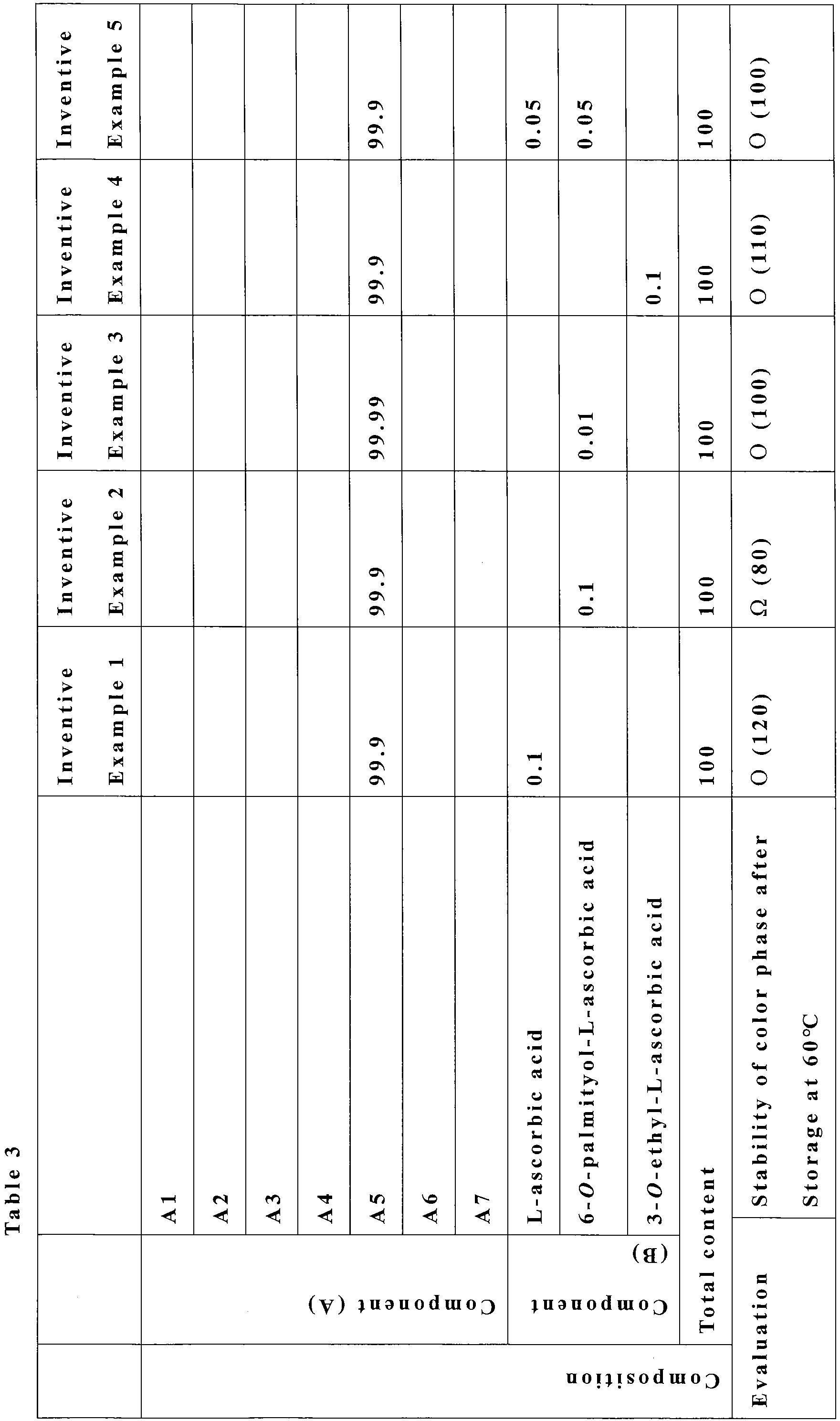

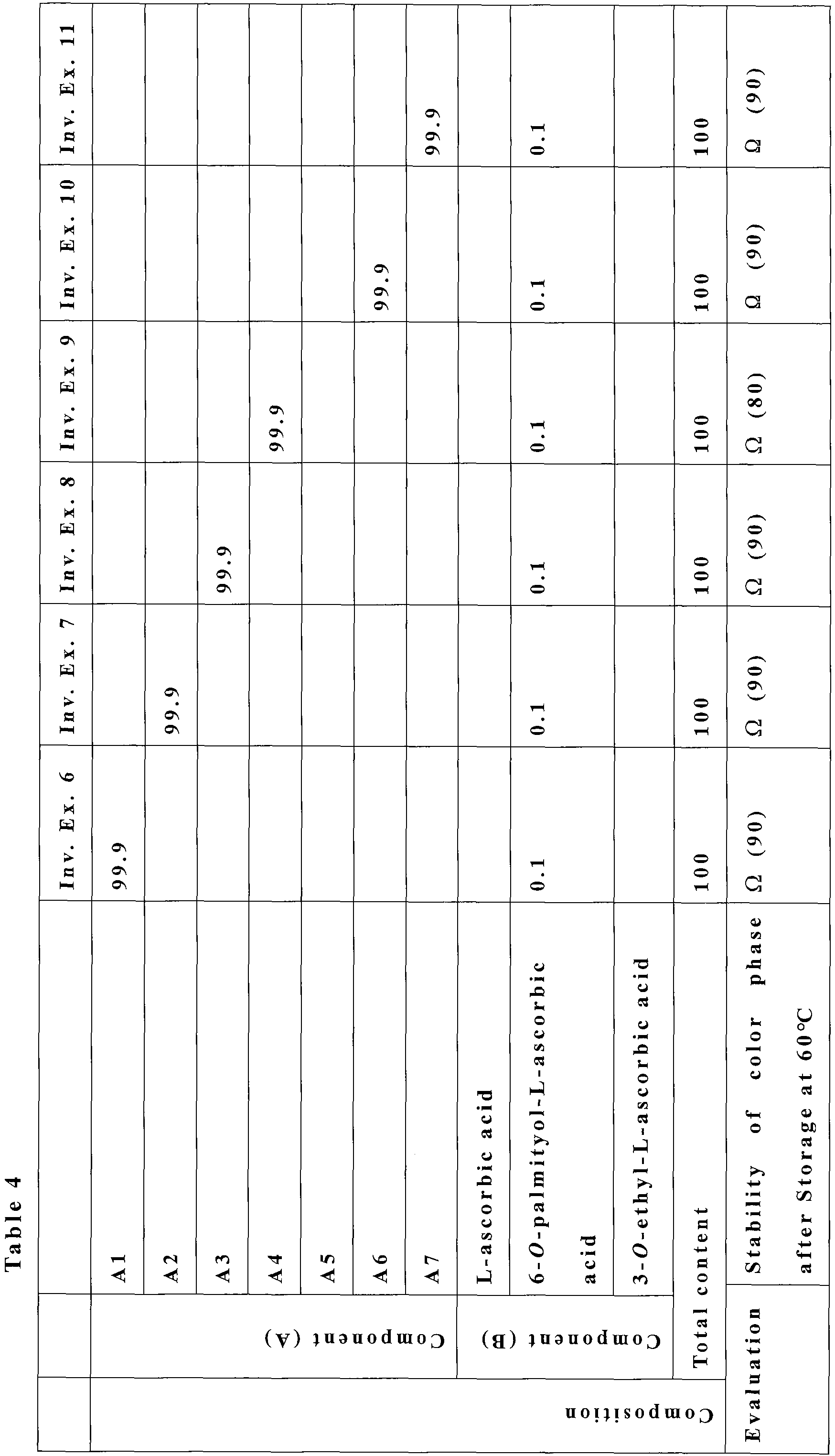

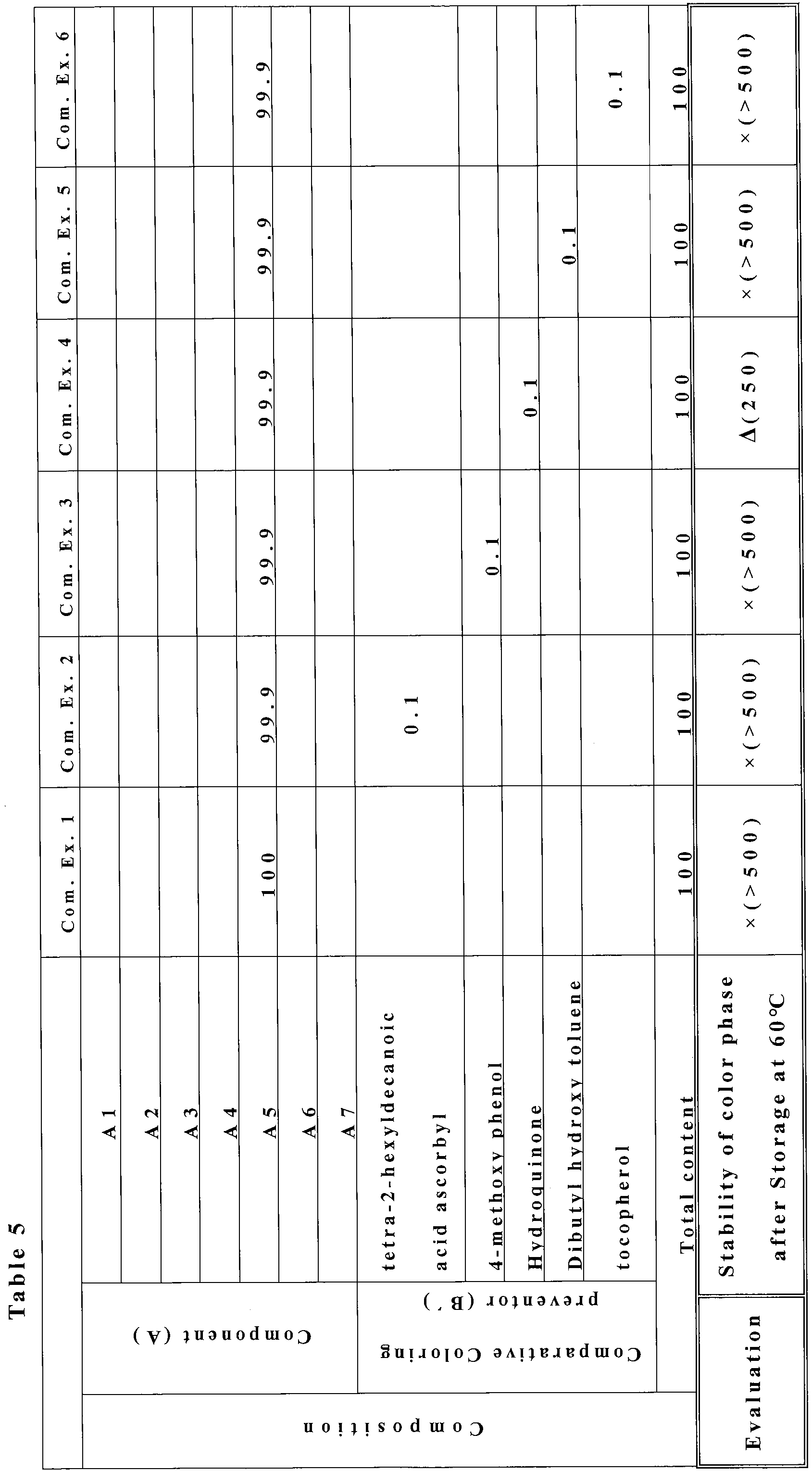

According to the results shown in tables 3 and 4, it was obtained the carbonate (meth)acrylate composition excellent in color phase in each of the inventive examples 1 to 11 of the present invention. Good result was obtained in the case that (2-oxo-1,3-dioxolan-4-yl) methyl methacrylate was applied as the carbonate (meth)acrylate (A) and 6-O-palmitoyl-L-ascorbic acid was applied as the ascorbic acid derivative (B) of the formula (1).

On the other hand, according to the comparative examples 1 to 6, as the ascorbic acid derivative (B) of the formula (1) was not added and a comparative coloring prevention agent (B') was added instead of it, the color phase of the carbonate (meth)acrylate composition after the storage at a high temperature was deteriorated.

### (Evaluation of polymerizability after storage)

As described in table 6, 200g of the carbonate (meth)acrylate compositions of the inventive example 2 and comparative example 1 were weighed and heated at 40°C for 1 week.

The carbonate (meth)acrylate compositions before the heating and after the heating were polymerized as follows. 180.0g of dimethyl formamide was charged in a 1-liter separable flask equipped with an agitator, thermometer, cooling machine, dropping funnel and nitrogen supply tube. The inside of the flask was replaced with nitrogen so that the inside was filled with nitrogen atmosphere. Monomer solution obtained by mixing 120.0g of the carbonate (meth)acrylate composition and 60.0g of dimethyl form amide and polymerization initiator solution obtained by mixing 2.2g of t-butyl peroxyneodecanoate and 40.0g of dimethyl form amide were prepared, respectively. The temperature in the inside of a reaction vessel was elevated to 75°C, and the monomer solution and polymerization initiator solution were added dropwise over 2 hours, respectively, at the same time, followed by the reaction at 75°C over 3 hours. The temperature was cooled to room temperature and 400.0g of polymerization solution was obtained.

Gel permeation chromatography (GPC) was applied to calculate the weight average molecular weights (Mw) of the respective polymerization products, under the following conditions.

The ratio of the change of Mw is defined as follows, and the polymerizability after the storage at a high temperature was evaluated based on the ratio of the change of Mw.
"Ω": 0% or higher and less than 5%
"O": 5% or higher and less than 10%
"×": 10% or higher
Ratio of change of Mw = (Mw of polymer product obtained from carbonate (meth)acrylate composition before heating minus (-) Mw of polymer product obtained from carbonate (meth)acrylate composition after heating) divided by (÷) Mw of polymer product obtained from carbonate (meth)acrylate composition before heating) × 100
System: "HLC-8320" manufactured by TOSOH CORPORATION
Column: "SB-805 HQ" manufactured by Shodex corporation
Standard substance: polystyrene
Eluent: DMF (dimethyl form amide)
Flow rate: 1.0ml/min
Temperature in column: 40°C
Detector: RI (differential refractive index detector)

**Table 6**

| Table 6 | | | |
|---|---|---|---|
| | | Inventive Example 2 | Comparative Example 1 |
| Polymerizability After storage at 40°C | Mw (before storage) | 200,000 | 199,000 |
| | Mw (after storage) | 196,000 | 149,000 |
| | Ratio of change of Mw | Ω (2) | × (25) |

According to the results shown in table 6, it was obtained the carbonate (meth)acrylate composition excellent in the polymerizability after the storage at a high temperature in the inventive example 2 of the present invention.

On the other hand, according to the comparative example 1, as the ascorbic acid derivative of formula (1) (component (B)) was not added and instead a comparative coloring inhibitor (component (B)) was added. As a result, Mw of the polymer product obtained from the carbonate (meth)acrylate composition after the storage at a high temperature was lower than that of the composition before the storage.

## Claims

1. A carbonate (meth)acrylate composition comprising 99 to 99.99 mass% of a carbonate (meth)acrylate and 0.01 to 1 mass% of an ascorbic acid derivative represented by a following formula (1), 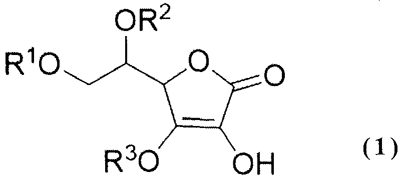 (In the formula (1),
R¹, R² and R³ independently represent hydrogen atom, alkyl group having a carbon number of 1 to 20 or acyl group having a carbon number of 1 to 20, respectively.).

2. The carbonate (meth)acrylate composition of claim 1, wherein said carbonate (meth)acrylate is represented by a following formula (2), formula (3) or formula (4), 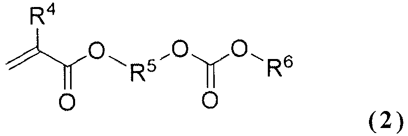 (In the formula (2),
R⁴ represents hydrogen atom or methyl group,
R⁵ represents alkylene group having a carbon number of 1 to 10, and
R⁶ represents hydrogen atom or alkyl group having a carbon number of 1 to 20.) 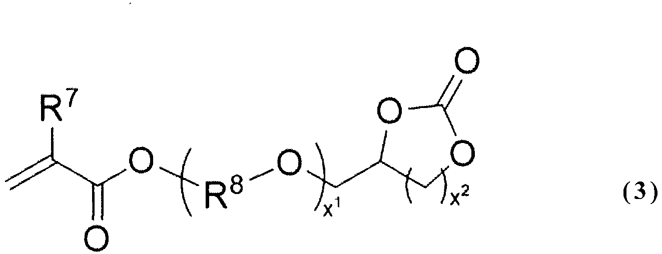 (In the formula (3),
R⁷ represents hydrogen atom or methyl group,
R⁸ represents alkylene group having a carbon number of 1 to 10,
X¹ represents 0 or 1, and
X² represents 0 to 2.) 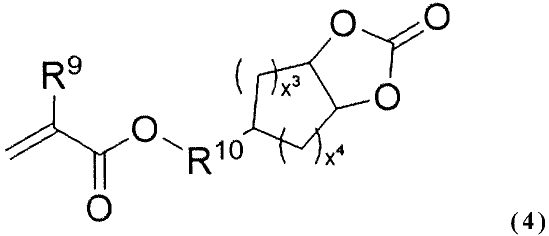 (In the formula (4),
R⁹ represents hydrogen atom or methyl group,
R¹⁰ represents alkylene group having a carbon number of 1 to 10,
X³ represents 0 to 2, and
X⁴ represents 0 to 1.).
